# FASCICULE DE BREVET EUROPEEN

(11) **EP 2 550 051 B1**
(45) Date de publication et mention de la délivrance du brevet: **23.10.2019**
(21) Numéro de dépôt: 11707883.2
(22) Date de dépôt: 01.02.2011
(51) Int. Cl.: A61M 16/10, A61M 16/00, F17D 1/04, A61G 13/10, A61G 12/00, F17C 13/08

(54) **INSTALLATION DE DISTRIBUTION DE GAZ MÉDICAUX AVEC DÉTECTION ET PILOTAGE AUTOMATIQUE EN CAS DE CHUTE DE PRESSION**
MEDIZINISCHE GASAUSGABEEINHEIT MIT AUTOMATISCHER ERKENNUNG UND KONTROLLE VON DRUCKABFALLEREIGNISSEN
MEDICAL GAS DISPENSING UNIT WITH AUTOMATIC DETECTION AND CONTROL IN THE EVENT OF A PRESSURE DROP

(30) Priorité: 25.03.2010 FR 1052145
(43) Date de publication de la demande: 30.01.2013
(73) Titulaire: Air Liquide Santé France, 75007 Paris (FR); L'Air Liquide Société Anonyme pour l'Etude et l'Exploitation des Procédés Georges Claude, 75007 Paris (FR)
(72) Inventeur: PITTET, François, F-92800 Puteaux (FR); PIEILLER, Paul, F-92800 Puteaux (FR)
(74) Mandataire: Pittis, Olivier
(86) Numéro de dépôt international: PCT/FR2011/050197
(87) Numéro de publication internationale: WO 2011/117488

(56) Documents cités:
- EP-A1- 1 977 712
- WO-A1-2006/121372
- FR-A1- 2 708 713
- US-A- 4 625 627
- US-A- 5 887 611

## Description

L'invention porte sur une installation de secours conçue pour garantir l'alimentation en fluides médicaux, en particulier en oxygène et en air, des réseaux de gaz médicaux équipant les services hospitaliers et analogues accueillant des patients hautement dépendants, en cas de dysfonctionnement de l'alimentation en gaz desdits réseaux, ainsi que le procédé de pilotage d'une telle installation.

Dans les bâtiments hospitaliers ou analogues, on utilise des réseaux de canalisations pour distribuer les fluides médicaux, en particulier l'oxygène, l'air médical et le vide, au sein des différentes pièces ou des différents services, tel que salles de soin, salles de réanimation, chambres..., dans lesquels ils sont utilisés pour le traitement des patients.

En cas de défaillance de l'alimentation en gaz ou en vide d'un réseau, il est connu de pouvoir remplacer temporairement la ou les sources de gaz ou de vide alimentant normalement ce réseau par une ou des sources de gaz ou de vide de secours.

Pour ce faire, on utilise des armoires fixes ou des unités mobiles de secours qui viennent se raccorder au réseau à secourir de manière à alimenter ledit réseau en gaz et/ou en vide.

Ainsi, le document EP-A-1977712 enseigne une unité fonctionnelle hospitalière mobile pour la distribution momentanée de fluides médicaux, comprenant une armoire montée sur roues renfermant plusieurs bouteilles de gaz médical, notamment de l'oxygène et de l'air médical, et une pompe d'aspiration du vide médical permettant de maintenir le réseau de canalisation en dépression. Des prises de distribution de fluide médical permettent de connecter l'unité au réseau de distribution de fluides médicaux d'un bâtiment hospitalier. Des moyens de surveillance et de pilotage sont prévus pour avertir d'un dysfonctionnement éventuel de l'unité.

Ce type d'unité de distribution de fluides permet de pallier toute défaillance de la distribution de fluides médicaux à partir du moment où cette défaillance a été détectée, par exemple si le personnel médical constate une chute de pression des gaz médicaux au niveau des prises utilisateurs ou une baisse de la dépression régnant dans le réseau de vide.

Cependant, si une telle unité est efficace et remplit parfaitement son rôle, elle ne permet pas de détecter automatiquement les défaillances du réseau de distribution de gaz médicaux agencé dans un bâtiment hospitalier, ni de pouvoir apporter une réponse automatique et rapide lorsqu'une telle défaillance se produit.

Or, comme on peut aisément l'imaginer, pouvoir réagir rapidement, voire immédiatement, en cas de rupture d'alimentation en gaz ou en vide est primordial dans certains services hospitaliers, notamment dans les salles d'urgence, de soins ou d'opération par exemple.

On connaît également le document FR-A-2708713 qui enseigne une installation de distribution de gaz, en particulier de gaz médicaux, tel l'oxygène et l'air médical, comprenant une ligne principale de gaz, une ligne annexe d'alimentation en gaz raccordée à la ligne principale de fluide, et comportant une vanne pneumatique apte à contrôler le passage de gaz depuis la ligne annexe vers ladite ligne principale, un dispositif anti-retour de gaz agencé sur la ligne principale de fluide, et des capteurs de pression. Le système de pilotage commande pneumatiquement la vanne pilotable.

En outre, le document US-A-4,625,627 enseigne une armoire contenant des bouteilles de gaz et des conduites de gaz reliées auxdites bouteilles pour acheminer des gaz. Elle est munie d'une porte pivotante avec une découpe donnant accès à l'intérieur de l'armoire. Le problème qui se pose est d'améliorer les unités ou installations de distribution de fluides médicaux, en particulier de gaz médicaux, tel l'oxygène ou l'air, de manière à pouvoir détecter automatiquement une défaillance du réseau de distribution de gaz médicaux au sein d'un hôpital ou analogue, et à pouvoir pallier automatiquement une telle défaillance et ce, préférentiellement en avertissant l'utilisateur, c'est-à-dire le personnel soignant, qu'une défaillance a eu lieu et qu'une procédure d'urgence a été mise en route.

La solution est une installation de distribution de gaz, en particulier de gaz médicaux, tel l'oxygène et l'air médical, comprenant :
- une ligne principale de gaz comprenant un orifice d'entrée et un orifice de sortie de gaz,
- une première ligne annexe d'alimentation en gaz raccordée fluidiquement, via une extrémité aval, à la ligne principale de fluide, et comportant au moins une vanne pilotable apte à contrôler le passage de gaz depuis la ligne annexe vers ladite ligne principale,
- un dispositif anti-retour de gaz agencé sur la ligne principale de fluide,
- un premier capteur de pression comportant une prise de pression gazeuse raccordée fluidiquement à la ligne principale entre l'orifice d'entrée et le moyen anti-retour, et/ou un deuxième capteur de pression comportant une prise de pression gazeuse raccordée fluidiquement à la ligne principale entre le moyen anti-retour et l'orifice de sortie, et
- un système de pilotage raccordé audit ou auxdits premier capteur de pression et/ou deuxième capteur de pression et commandant ladite au moins une vanne pilotable en réponse à au moins une mesure de pression opérée par l'un au moins desdits capteurs,
ladite installation étant caractérisée en ce que :
- la vanne pilotable est une électrovanne,
- le système de pilotage comporte au moins un automate programmable,
- au moins une partie de la ligne principale de fluide, la ligne annexe d'alimentation en fluide, la vanne pilotable, le dispositif anti-retour, le premier capteur de pression, le deuxième capteur de pression et le système de pilotage sont compris dans une armoire,
- et elle comporte en outre :
   - une ligne de bipasse reliée fluidiquement à la première ligne annexe d'alimentation, en amont et aval de la vanne pilotable, ladite ligne de bipasse comprenant une vanne manuelle manœuvrable par l'utilisateur qui, en temps normal, est fermée, et
   - un boîtier à panneau de contrôle relié au système de pilotage, pour afficher une alarme spécifique pour avertir l'utilisateur, en cas de dysfonctionnement de l'électrovanne, et lui permettre d'actionner et ouvrir manuellement la vanne manuelle.

Selon le cas, l'installation de l'invention peut comprendre l'une ou plusieurs des caractéristiques suivantes :
- elle comporte en outre au moins une bouteille de gaz raccordée fluidiquement à une extrémité amont de la ligne annexe d'alimentation en gaz, en particulier une ou plusieurs bouteilles d'oxygène et/ou d'air médical.
   - le système de pilotage comporte plusieurs automates programmables.
- le dispositif anti-retour comprend un clapet anti-retour.
- la première ligne annexe d'alimentation en gaz comprend un dispositif de réduction de pression agencé entre la bouteille et la vanne pilotable.
- elle comporte une deuxième ligne annexe d'alimentation en gaz reliée fluidiquement par son extrémité amont à une bouteille de gaz et par son extrémité aval à la ligne principale de fluide, de préférence entre le moyen anti-retour et l'orifice de sortie.
- elle comporte une alarme sonore ou visuelle se déclenchant en réponse à une détection par l'un ou l'autre des capteurs d'une valeur de pression inférieure ou égale à une valeur de pression seuil au sein de la ligne principale de gaz.
- les capteurs sont aptes à et conçus pour mesurer la pression instantanée régnant dans la ligne, en amont ou en aval du clapet anti-retour, et à transmettre ces mesures de pression sous forme de signaux électriques au système de pilotage.
- une découpe dans l'une des portes de l'armoire permet à l'utilisateur d'avoir accès à différents afficheurs ou touches de commande, situés sur le boîtier à panneau de contrôle.
- l'armoire est fixe, c'est-à-dire non mobile.

L'invention porte aussi sur une canalisation d'acheminement d'un gaz entre une source de gaz et au moins un site utilisateur, ladite source de gaz étant raccordée fluidiquement à une entrée de ladite canalisation de manière alimenter ladite canalisation avec ledit gaz, caractérisée en ce que ladite canalisation est raccordée en outre au moins à une entrée et à au moins une sortie d'une ligne principale de gaz d'une installation selon l'invention.

De préférence, l'installation selon l'invention est agencée sur ladite canalisation en aval d'une unité de détende de gaz elle-même agencée sur ladite canalisation.

Par ailleurs, le pilotage d'une installation selon l'invention agencée sur une canalisation de distribution de gaz selon l'invention, est opéré selon les étapes de :
a) déterminer au moyen d'au moins l'un desdits premier et deuxième capteurs, la pression instantanée de gaz régnant dans au moins une ligne principale de l'installation de distribution de gaz,
b) comparer au moyen du système de pilotage, la pression instantanée déterminée à l'étape a) avec au moins une valeur-seuil de pression préfixée,
c) commander au moyen du système de pilotage, une ouverture de la vanne pilotable lorsqu'à l'étape b), on détermine que la pression instantanée mesurée est inférieure à la valeur-seuil de pression, et alimenter au moins une partie de la ligne principale et de la canalisation reliée fluidiquement à ladite ligne principale avec du gaz à une pression supérieure à ladite valeur-seuil de pression, issu d'une bouteille de gaz de l'installation.

Selon le cas, ce procédé de pilotage peut comprendre l'une ou plusieurs des caractéristiques suivantes :
- à l'étape c) on commande une ouverture de la vanne pilotable, lorsque la pression instantanée atteint une valeur de pression critique inférieure à ladite valeur-seuil de pression.
- lorsqu'à l'étape b), on détermine que la pression instantanée mesurée est inférieure à la valeur-seuil de pression, on déclenche au moins une alarme sonore ou visuelle, ou les deux.
- le gaz est de l'oxygène ou de l'air, notamment de l'air de qualité médicale.
- le procédé et l'installation sont mise en œuvre au sein d'un bâtiment hospitalier.

La présente invention va maintenant être décrite plus en détail en références aux Figures annexées parmi lesquelles :
- la Figure 1 représente une vue schématique d'une installation de distribution de fluides médicaux selon l'invention agencée sur le réseau d'oxygène d'un bâtiment hospitalier,
- la Figure 2 montre l'installation de la Figure 1 raccordée au réseau d'air médical du bâtiment hospitalier,
- la Figure 3 est un schéma de principe du mode de fonctionnement de l'installation des Figures 1 et 2,
- la Figure 4 représente une armoire d'une installation selon l'invention,
- la Figure 5 schématise un boîtier d'alarme utilisable dans le cadre de la présente invention, et
- la Figure 6 schématise l'interface homme/machine d'un automate programmable utilisable dans le système de pilotage d'une installation selon l'invention dans le cadre de la présente invention.

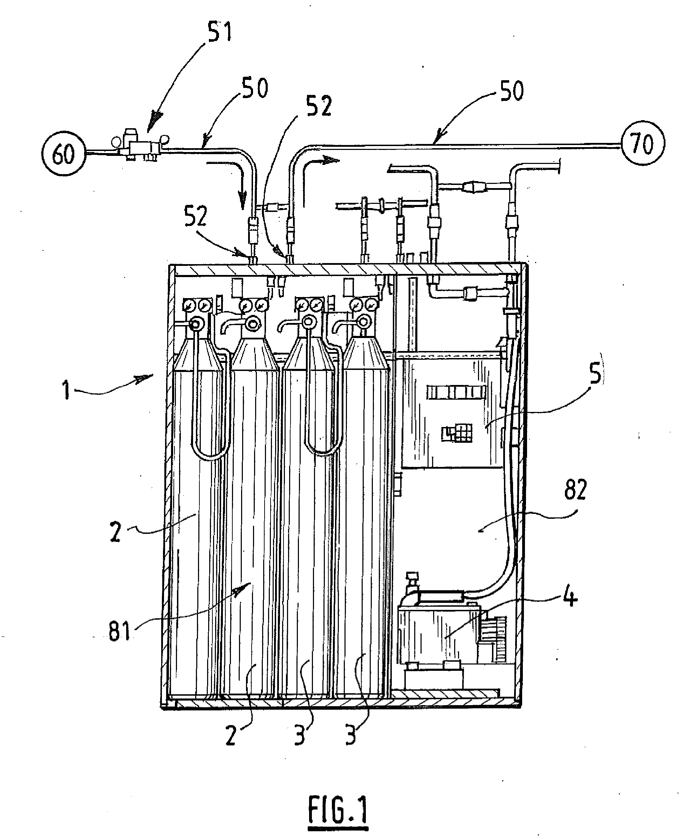

La Figure 1 représente une vue schématique d'une installation de distribution de fluides médicaux selon l'invention comprenant une armoire 1 fixe reliée au réseau de canalisations 50 d'oxygène acheminant l'oxygène médical au sein d'un bâtiment hospitalier depuis une (ou plusieurs) source d'oxygène médical 60, typiquement un réservoir d'oxygène généralement situé à l'extérieur du ou des bâtiments, et un ou des sites utilisateurs d'oxygène 70, en particulier une ou plusieurs salles d'opération, salles de soins, salles de réanimation, chambres etc...

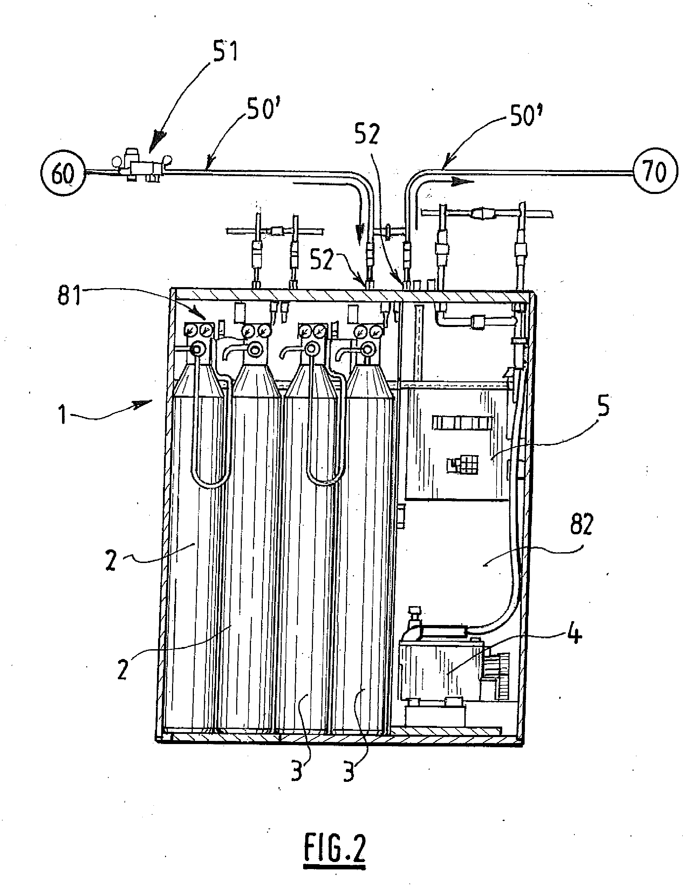

De manière analogue, la Figure 2 représente l'armoire 1 de la Figure 1 qui est également reliée au réseau de canalisations 50' distribuant l'air de qualité médicale au sein du bâtiment considéré. Là encore, la source 60' d'air médical est typiquement un réservoir d'air médical sur site ou un mélangeur de gaz (mélange O₂/N₂), généralement situé à l'extérieur du ou des bâtiments.

L'air ou l'oxygène médicaux peuvent être produits sur site, par exemple au moyen d'unités d'adsorption à pression ou vide modulé de type PSA (pressure swing adsorption) ou VSA (vacuum swing adsorption), ou alors acheminés sur site par camion citerne par exemple.

Comme on le voit sur les Figures 1 et 2, l'armoire 1 comporte, en partie gauche 81, des bouteilles de gaz type B50 contenant des gaz médicaux à une pression pouvant aller jusqu'à 300 bar, à savoir deux bouteilles 2 contenant de l'oxygène et deux bouteilles 3 contenant de l'air médical, et en partie droite 82, une pompe à vide 4 reliée au réseau de vide du bâtiment hospitalier, ainsi qu'un système de pilotage 5 apte à et conçu pour piloter la distribution des gaz provenant des bouteilles 2, 3, comme détaillé ci-après, ou du vide produit par la pompe à vide 4.

A titre d'exemple, on peut utiliser une pompe à vide 4 de 40 m³/h reliée au réseau de vide du bâtiment. Le contrôle de la pompe à vide 4 se faisant d'une manière différente de celui de l'alimentation en oxygène et en air, il ne sera pas détaillé ci-après.

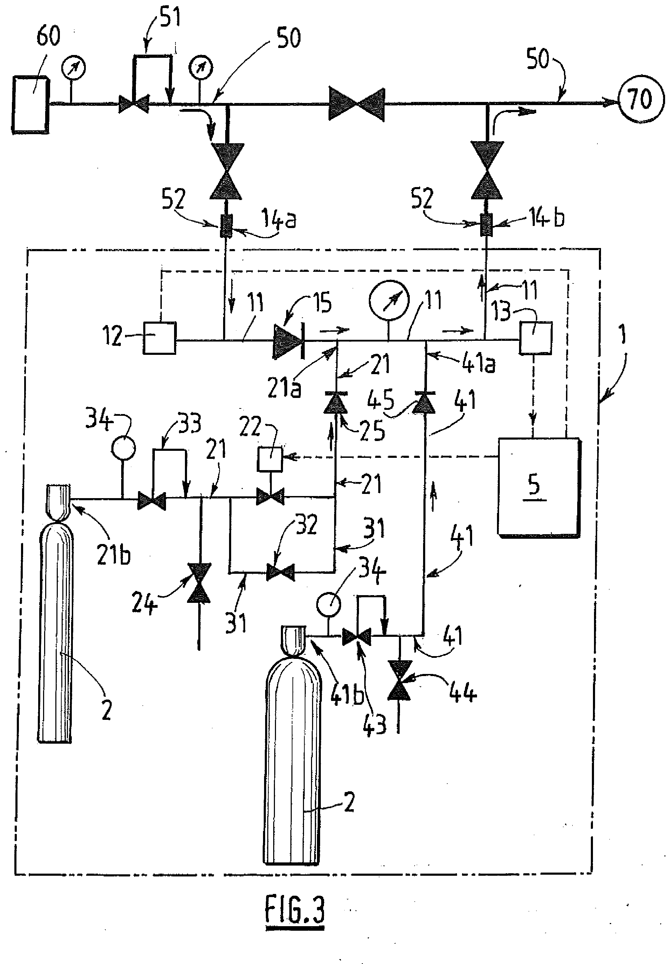

Les bouteilles 2, 3 sont équipées de manière classique de robinets équipés de détendeurs en sortie 33, 43 (Fig. 3) et de manomètres 34 indicateurs de pression permettant de vérifier et contrôler la pression du gaz en sortie des bouteilles 2, 3. Par exemple, on peut utiliser des bouteilles de type B50 contenant environ 10 m³ de gaz de manière à obtenir un débit maximal de l'ordre de 30 m³/h.

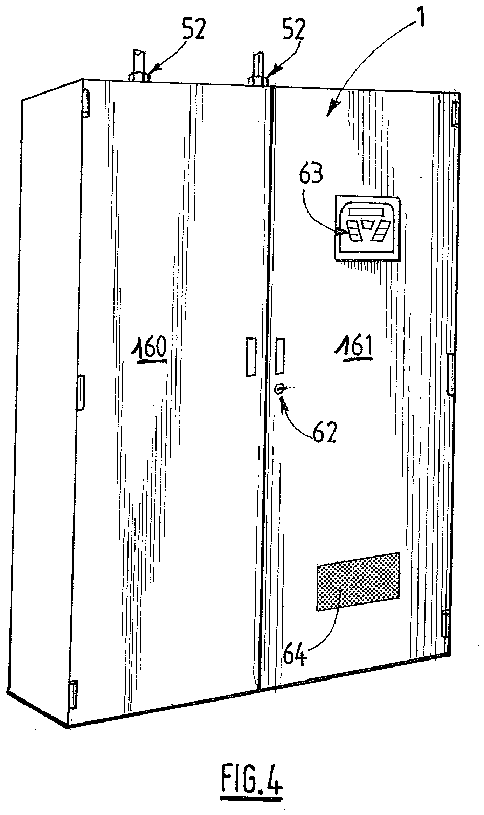

Comme illustré sur la Figure 4, l'armoire 1 est munie de portes 160, 161 munies d'un système de verrouillage 62 classique, c'est-à-dire une serrure à clé ou analogue, permettant de protéger les différents éléments et systèmes qu'elle renferme de la poussière et/ou de manipulations inopinées ou par des personnes non autorisées.

Par ailleurs, une découpe dans l'une 161 des portes permet à l'utilisateur d'avoir accès à différents afficheurs, touches de commande ou analogues situés sur un boîtier 63 à panneau de contrôle relié au système de pilotage 5, en particulier on peut utiliser un boîtier de type VIGI 3077 commercialisé par la société Air Liquide Medical Systems qui reçoit toutes les informations des automates du système de pilotage 5 et qui affiche les alarmes destinées aux utilisateurs, par exemple des informations générales de fonctionnement.

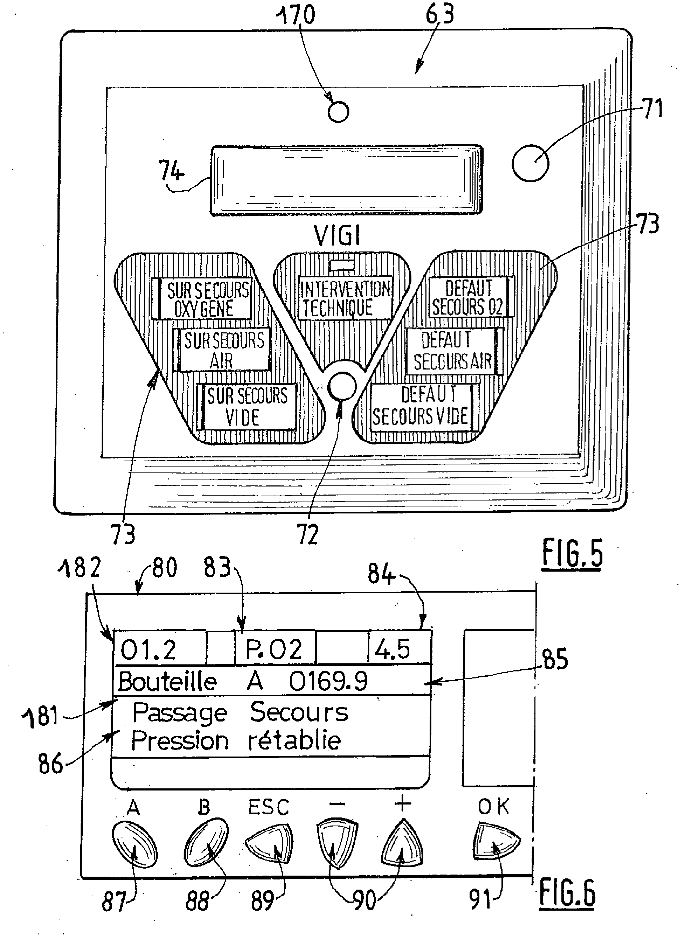

Comme visible sur la Figure 5, un boîtier 63 de type VIGI 3077 comporte un voyant 170 de bon fonctionnement (voyant vert), un bouton ou touche d'acquittement 71 pour inhiber temporairement une alarme sonore, un bouton ou touche 72 de test pour l'afficheur, les voyants, etc..., des voyants d'alarmes 73 qui clignotent en cas de défaut et un écran d'affichage 74 des différentes alarmes.

Les informations peuvent également être affichées à distance, dans les salles de soins ou analogues, par l'intermédiaire de boîtiers de report d'alarmes, par exemple de type VIGI 3003, également commercialisé par Air Liquide Medical Systems.

Une armoire 1 selon l'invention peut avoir une hauteur d'environ 1,5 à 2 m, une largeur d'environ 1,20 à 1,70 m et une profondeur d'environ 0,35 à 0,70 m. Par exemple, l'armoire 1 représentée sur la Figure 4 a une hauteur de 1,85 m, une largeur de 1,55 m et une profondeur de 0,44 m.

La coque externe formant l'armoire 1 est formée d'une tôle en acier avec un revêtement de peinture et comporte une ou plusieurs ouvertures 64, préférentiellement munies de grilles ou analogues, servant à l'aération de l'intérieur de l'armoire 1, comme illustré en Figure 4.

L'armoire 1 permet d'assurer un secours en fluides médicaux directement sur les réseaux secondaires, c'est-à-dire sur la partie des réseaux 50, 50' situés en aval d'unités de détente 51 généralement agencées sur les canalisations d'oxygène et d'air au sein des bâtiments hospitaliers, en considérant le sens de circulation des gaz depuis leurs sources 60, 60' jusqu'aux sites utilisateurs 70 ; la partie de réseau située en amont de chaque unité de détente 51 étant appelée réseau primaire.

De cette manière, l'armoire 1 de secours réalimente uniquement la partie du réseau concernée par la défaillance, c'est-à-dire schématiquement les parties de canalisations situées entre l'armoire 1 et les sites utilisateurs 70, donc uniquement le réseau secondaire.

En tant qu'unité de détente, on peut utiliser le dispositif appelé DAMAO commercialisé par Air Liquide Medical Systems permettant d'obtenir une pression de détente normale de 4,8 bar par exemple dans les réseaux d'oxygène 50 et d'air 50'.

Pour les gaz comprimés, à savoir l'air et l'oxygène, le secours du réseau secondaire est assuré automatiquement à la pression normale d'utilisation avec un secours « ultime » à une pression inférieure à la pression normale de service, comme il sera expliqué ci-après.

Dans tous les cas, comme illustré en Figure 3, l'installation de distribution de gaz de l'invention comporte, agencés dans l'armoire 1, une ligne principale de gaz 11 comprenant un orifice d'entrée 14a et un orifice de sortie 14b de gaz. De préférence, ces orifices 14a, 14b sont situés au niveau des extrémités de la ligne principale 11 qui comprennent des raccords 52 permettant la connexion de ladite ligne principale 11 aux canalisations 50, 50' du réseau de canalisations d'oxygène et d'air médical qui acheminent ces gaz dans le bâtiment hospitalier.

L'armoire 1 peut aussi être équipée d'une rampe d'accès facilitant la mise en place des bouteilles 2, 3.

Dans un souci de simplification et éviter des répétitions inutiles, seule la partie de l'installation selon l'invention distribuant l'oxygène médical sera détaillée ci-après, en références aux Figures 1 et 3. Toutefois, il est à préciser que la partie de l'installation distribuant l'air médical (Fig. 2) est organisée et fonctionne exactement de la même façon et sur le même principe ; cette dernière fait donc également partie de la présente invention bien que non détaillée dans la présente description.

Comme illustré en Figure 3, pour assurer le secours en oxygène (ou en air) en cas de défaillance du réseau 50, une première ligne annexe 21 d'alimentation en gaz, telle une canalisation souple, est raccordée fluidiquement, via son extrémité amont 21b, à une bouteille d'oxygène 2 et, via son extrémité aval 21a, à la ligne principale de fluide 11 de manière à pouvoir y distribuer de l'oxygène de secours.

La circulation de l'oxygène au sein de la ligne annexe 21 depuis la bouteille 2 à la ligne principale 11 est contrôlée par une vanne pilotable 22, en particulier une électrovanne. On peut par exemple utiliser une électrovanne de type 6027 2/2 NF commercialisée par la société BURKERT.

Cette vanne 22 pilotable est commandée à distance par le système de pilotage 5, par exemple un ou plusieurs automates programmables avec calculateur interne, notamment de référence commerciale Millenium 3 commercialisés par la société Crouzet.

L'interface homme/machine 80 d'un tel automate constituant tout ou partie du système de pilotage 5 est schématisée en Figure 6. On peut voir qu'elle comprend un afficheur 181 apte à afficher différentes informations, tel que la pression en amont 182 et en aval 84 de l'armoire 1, la nature 83 du gaz concerné, la pression résiduelle 85 dans la bouteille 2 de secours considérée, des messages 86 d'alerte ou d'état de l'installation, ainsi que différentes touches activables par l'utilisateur, notamment une touche (A) de défilement de seuils 87, une touche (B) 88 de lancement de tests (avec touche -) ou de modification de seuils (avec touche +), une touche 89 d'interruption (ESC) de mode test, des touches 90 de réglage de seuils (+) et (-), et une touche 91 d'acquittement pour la validation des modifications de seuils de pression.

En outre, un dispositif anti-retour de gaz 15, tel d'un clapet anti-retour, est agencé sur la ligne principale 11 de fluide. Celui-ci permet d'éviter des remontées de gaz provenant de la bouteille 2, c'est-à-dire ici d'oxygène, vers l'unité de détente 51 lorsque la bouteille 2 alimente la ligne principale 11.

La surveillance de la pression dans la ligne 11 est opérée au moyen de deux capteurs de pression 12, 13. On peut par exemple utiliser des capteurs de type 21SR commercialisés par la société KELLER.

Plus précisément, on utilise un premier capteur de pression 12, encore appelé capteur amont 12, dont la prise de pression gazeuse est raccordée fluidiquement à la ligne principale 11 entre l'orifice d'entrée 14a et le moyen anti-retour 15, et un deuxième capteur de pression 13, encore appelé capteur aval 13, dont la prise de pression gazeuse est raccordée fluidiquement à la ligne principale 11 entre le moyen anti-retour 15 et l'orifice de sortie 14b.

Ces capteurs 12, 13 permettent de mesurer, soit en permanence, soit à des intervalles de temps donnés, la pression instantanée régnant dans la ligne 11, en amont ou en aval du clapet anti-retour 15, et ensuite de transmettre ces mesures de pression sous forme de signaux électriques au système de pilotage 5.

Au sein du système de pilotage 5, les signaux correspondant aux mesures de pression instantanée des capteurs sont comparés à des valeurs seuils prédéterminées et en fonction du résultat de ces comparaisons, la vanne pilotable 22 est commandée soit pour rester fermée si aucun dysfonctionnement n'est détecté et que le niveau de pression gazeuse qui règne dans la ligne principale 11 est considéré comme étant normal, soit pour s'ouvrir et laisser passer de l'oxygène en direction de la ligne principale 11 si une chute de pression est détectée.

Le système de pilotage 5 et les capteurs 12, 13 notamment sont alimentés en courant électrique par le secteur, c'est-à-dire fonctionnement sur 220 V et 16 A. Toutefois, faire fonctionner l'installation à un autre voltage et/ou ampérage est tout à fait possible moyennant quelques adaptations de routine, notamment via l'utilisation de transformateurs ou convertisseurs de courant.

Le système de pilotage 5 commande donc la vanne 22 en réponse aux mesures de pression opérées par l'un au moins des capteurs 12, 13. En cas de détection d'un niveau de pression inapproprié, une alarme sonore et/ou visuelle peut être déclenchée pour avertir le personnel soignant du problème rencontré.

Il est à noter qu'on prévoit par ailleurs une ligne de bipasse 31 reliée fluidiquement à la première ligne annexe d'alimentation 21, en amont et aval de la vanne pilotable 22, de manière à pouvoir bipasser la vanne pilotable 22, notamment en cas de défaillance de celle-ci, et ainsi alimenter le réseau 50 avec de l'oxygène. Cette ligne de bipasse 31 comprend une vanne manuelle 32 manœuvrable par l'utilisateur qui, en temps normal, est fermée pour ne pas laisser d'oxygène transiter par cette ligne de bipasse 31.

Par ailleurs, on prévoit une deuxième ligne annexe d'alimentation en gaz 41 reliée fluidiquement par son extrémité amont 41b à une bouteille de gaz 2, à savoir d'oxygène ici, et par son extrémité aval 41a à la ligne principale de fluide 11, de préférence entre le moyen anti-retour 15 et l'orifice de sortie 14b. Cette deuxième ligne annexe 41 permet d'alimenter le réseau 50 soit en cas de défaillance de la première ligne annexe 21, soit lorsque la bouteille d'oxygène 2 alimentant ladite première ligne annexe 21 est vide.

La première ligne annexe 21 et la deuxième ligne annexe 41 comprennent chacune un dispositif de purge 24, 44 servant à purger le gaz contenu dans ces lignes 21, 41, notamment lors d'opérations de maintenance ou de remplacement de bouteilles, ainsi que des clapets anti-retours supplémentaires 25, 45 permettant d'empêcher des remontées de gaz provenant de la ligne principale 11 ou du réseau 50 vers les bouteilles 2.

L'installation de l'invention fonctionne schématiquement de la manière suivante pour ce qui est de la surveillance du réseau d'oxygène (ou d'air).

En l'absence de défaillance du réseau 50 d'oxygène, la pression dans le réseau 50, en particulier dans la ligne principale 11 est suffisante, par exemple une pression entre 3,8 et 4,8 bar, et l'armoire 1 ne déclenche aucune alarme. Le clapet anti-retour 15 est en position ouverte et l'oxygène gazeux s'écoule normalement dans le réseau secondaire et au travers de la ligne principale 11 depuis son orifice d'entrée 14a en direction de son orifice de sortie 14b, puis des sites utilisateurs 70 d'oxygène situés aux extrémités du réseau secondaire lui-même situé en aval de chaque unité de détente 51.

Par contre, si la pression d'oxygène au sein du réseau 50 secondaire chute en dessous d'une valeur-seuil de 3,8 bar par exemple, alors le ou les capteurs 12, 13 détectent cette chute de pression et un signal de mesure de pression correspondant est transmis au système de pilotage 5 qui déclenche alors la séquence suivante:
- à 3,8 bar par exemple, le système de pilotage 5 déclenche une alarme sonore et/ou visuelle pour informer les utilisateurs d'un passage imminent sur les secours, c'est-à-dire que de l'oxygène provenant de la première bouteille 2 va être utilisée pour alimenter la partie secondaire du réseau 50. Ces signaux d'alarme s'affichent sur le boîtier VIGI 3077 de la Figure 5, comme expliqué ci-avant.
- à 3,5 bar par exemple, si la pression continue de chuter, le capteur amont 14a commande, via le système de pilotage 5, une ouverture de l'électrovanne 22, ce qui autorise alors la circulation d'oxygène à une pression d'au moins 4,5 bar de la bouteille 2 à la ligne principale 11, puis vers le réseau 50 secondaire. Le détendeur 33 est quant à lui réglé à une pression d'oxygène d'au moins 4,2 bar, typiquement de l'ordre de 4,5 bar. Le clapet anti-retour 15 passe alors en position fermée, ce qui empêche les remontées d'oxygène sous pression vers l'unité de détente 51. Le passage effectif sur l'oxygène de secours provenant de la première bouteille 2 peut aussi faire l'objet d'un déclenchement d'alarme sonore ou visuelle visant à avertir les utilisateurs.
- l'oxygène sous pression remplit donc les canalisations 50 du réseau secondaire, ce qui provoque une remontée du niveau de pression dans ces canalisations situées en aval de l'armoire 1. En cas de panne de l'électrovanne 22 qui resterait alors en position fermée, une alarme spécifique se déclenche pour avertir l'utilisateur et lui permettre d'actionner et ouvrir manuellement la vanne manuelle 32 agencée sur la ligne de bipasse 31.
- quand la durée d'utilisation de la première bouteille 2 dépasse une durée préfixée ou que la pression au sein de ladite bouteille 2 chute en dessous d'une valeur minimale, par exemple 30 bar, alors une alarme spécifique alerte l'utilisateur d'un passage imminent sur la deuxième bouteille d'oxygène 2 et de la nécessité de remplacer la première bouteille 2 vide par une nouvelle bouteille 2 pleine d'oxygène. Lorsque la première bouteille 2 est totalement vide ou presque, la deuxième bouteille 2 prend automatiquement le relais en délivrant de l'oxygène à une pression inférieure ou égale à la valeur normale, en aval de l'armoire 1, par exemple de l'oxygène à 3,2 bar, via la deuxième ligne annexe d'alimentation en gaz 41 reliant la deuxième bouteille 2 à la ligne principale 11.
- dans tous les cas, le système de pilotage 5 ordonne une fermeture de l'électrovanne 22, lorsque la pression d'oxygène dans la ou les canalisations du réseau secondaire redevient supérieure ou égale à la valeur-seuil, par exemple 3,8 bar. Si un défaut d'électrovanne 22 a eu lieu durant le fonctionnement, comme évoqué ci-avant, alors une alarme spécifique signale à l'utilisateur qu'il doit actionner la vanne manuelle 32 pour fermer la ligne de bipasse 31.

Dans le cadre de la présente invention, le système de pilotage 5 commande une ouverture de la vanne pilotable 22 immédiatement après avoir déterminé que la pression instantanée mesurée par le ou les capteurs de pression 12, 13 est inférieure à la valeur-seuil de pression préfixée, par exemple 3,8 bar, ou alors, selon le cas, uniquement si la pression continue à chuter et atteint une valeur de pression critique inférieure à ladite valeur-seuil de pression, par exemple une valeur de pression critique de 3,5 bar. Dans le deuxième cas, les valeurs de pression (valeur-seuil de pression et pression critique) constituent en quelque sorte des paliers de pression. Ces valeurs de pressions sont mémorisées et/ou enregistrées au sein du système de pilotage 5.

Comme expliqué ci-avant, l'installation selon l'invention comporte un deuxième capteur 13 de pression, appelé capteur aval. Celui-ci sert, en cas de dysfonctionnement du premier capteur 12 ou capteur amont, à transmettre les informations de pression au système de pilotage 5. Le pilotage de l'armoire 1 est donc réalisé dans ce cas exclusivement grâce aux signaux transmis par le capteur aval 13.

Dans ce cas, le dysfonctionnement du capteur amont 12 est signalé par une alarme ; le capteur amont 12 n'étant lui-même plus apte à déclencher une alarme en cas de chute de pression.

Si la pression mesurée par le capteur aval 13 est correcte dans le réseau secondaire et dans la ligne principale 11, alors rien d'autre ne se passe.

Par contre, si une chute de pression est observée par le capteur 13, alors la séquence est similaire à la séquence précédente mis à part qu'à 3,5 bar de pression, c'est le capteur aval 13 qui ordonne, via le système de pilotage 5, l'ouverture de l'électrovanne 22 mais seulement pendant une durée limitée préfixée, typiquement pendant quelques minutes, par exemple 5 minutes. Le réglage de la temporisation, c'est-à-dire de la durée de commande d'ouverture, se fait au niveau du système de pilotage 5.

Si après cette durée préfixée, le défaut responsable de la chute de pression dans le réseau 50 a disparu, alors l'installation repasse en veille. Dans le cas contraire, la séquence est répétée de manière cyclique, autant de fois que nécessaire.

De la même manière, tout défaut du capteur aval 13 est signalé par une alarme. En cas de chute simultanée de pression, celle-ci est détectée par le capteur 12 amont qui déclenche non seulement une alarme mais également la séquence décrite précédemment avec ouverture de l'électrovanne 22, avertissement de l'utilisateur du passage sur l'oxygène de secours et, dans ce cas, l'obligation de vérifier la pression en aval de l'armoire 1 sur un manomètre de contrôle grâce au déclenchement d'une alarme spécifique de contrôle de pression.

Il est bon de rappeler à ce stade que le contrôle de la pression du réseau de canalisations 50' de la Figure 2 acheminant l'air de qualité médicale depuis le réseau primaire vers le réseau secondaire en air médical est opéré exactement de la même manière et avec les mêmes dispositifs et autres systèmes que ceux utilisés pour contrôler la pression en oxygène et qui ont été détaillés ci-avant. Les séquences et modes de fonctionnement décrits ci-dessus pour l'oxygène s'appliquent donc, dans le cadre de la présente invention, stricto sensu et de la même façon à la surveillance et à la régulation de la pression en air médical, en mettant en œuvre des bouteilles 3 d'air médical de secours en lieu et place des bouteilles 2 d'oxygène médical.

Les niveaux de pression d'air ou d'oxygène peuvent être identiques ou différents selon le fluide considéré. Ainsi, le tableau ci-dessous donne des exemples de valeurs de pression utilisables pour programmer le système de pilotage 5 en fonction du gaz considéré, à savoir oxygène (O₂) ou air de qualité médicale.

**Tableau**

| Pressions utilisables pour : | O₂ (en bar) | Air (en bar) |
|---|---|---|
| Réglage de l'unité de détente | 4,8 | 4,5 |
| Alarme passage sur gaz de secours | 3,8 | 3,6 |
| Pression de déclenchement gaz de secours | 3,5 | 3,3 |
| Réglage détendeur de la première bouteille | 4,5 | 4,2 |
| Réglage détendeur de la deuxième bouteille | 3,2 | 3 |
| Alarme défaut de la ligne de secours | 3 | 2,8 |
| Alarme de pression haute | 5,8 | 5,4 |

L'armoire 1 de l'invention permet de réaliser automatique un secours efficace et rapide, pendant quelques heures, d'un réseau de distribution d'oxygène et/ou d'air médical au sein d'un bâtiment hospitalier et permettre ainsi, par exemple qu'une opération chirurgicale ou similaire puisse se terminer normalement, même en cas de défaillance ou de panne du réseau primaire de canalisations alimentant normalement le bâtiment hospitalier.

## Revendications

1. Installation de distribution de gaz comprenant :
- une ligne principale de gaz (11) comprenant un orifice d'entrée (14a) et un orifice de sortie (14b) de gaz,
- une première ligne annexe d'alimentation en gaz (21) raccordée fluidiquement, via une extrémité aval (21a), à la ligne principale de fluide (11), et comportant au moins une vanne pilotable (22) apte à contrôler le passage de gaz depuis la ligne annexe (21) vers ladite ligne principale (11),
- un dispositif anti-retour de gaz (15) agencé sur la ligne principale (11) de fluide,
- un premier capteur de pression (12) comportant une prise de pression gazeuse raccordée fluidiquement à la ligne principale (11) entre l'orifice d'entrée (14a) et le moyen anti-retour (15), et/ou un deuxième capteur de pression (13) comportant une prise de pression gazeuse raccordée fluidiquement à la ligne principale (11) entre le moyen anti-retour (15) et l'orifice de sortie (14b), et
- un système de pilotage (5) raccordé audit ou auxdits premier capteur de pression (12) et/ou deuxième capteur de pression (13) et commandant ladite au moins une vanne pilotable (22) en réponse à au moins une mesure de pression opérée par l'un au moins desdits capteurs (12, 13),
**caractérisée en ce que** :
- la vanne pilotable (22) est une électrovanne,
- le système de pilotage (5) comporte au moins un automate programmable,
- au moins une partie de la ligne principale de fluide (11), la ligne annexe d'alimentation en fluide (21), la vanne pilotable (22), le dispositif anti-retour, le premier capteur de pression (12), le deuxième capteur (13) de pression et le système de pilotage (5) sont compris dans une armoire (1),
- et elle comporte en outre :
. une ligne de bipasse (31) reliée fluidiquement à la première ligne annexe d'alimentation (21), en amont et aval de la vanne pilotable (22), ladite ligne de bipasse (31) comprenant une vanne manuelle (32) manœuvrable par l'utilisateur qui, en temps normal, est fermée, et
. un boîtier (63) à panneau de contrôle relié au système de pilotage (5),pour afficher une alarme spécifique pour avertir l'utilisateur, en cas de dysfonctionnement de l'électrovanne (22), et lui permettre d'actionner et ouvrir manuellement la vanne manuelle (32).

2. Installation selon la revendication précédente, **caractérisée en ce qu'**elle comporte en outre au moins une bouteille (2) de gaz raccordée fluidiquement à une extrémité amont (21b) de la ligne annexe (21) d'alimentation en gaz.

3. Installation selon la revendication 1, **caractérisée en ce que** le système de pilotage (5) commande un déclenchement de l'alarme spécifique avertissant l'utilisateur en cas de dysfonctionnement de l'électrovanne (22).

4. Installation selon au moins l'une des revendications précédentes, **caractérisée en ce que** le boîtier (63) à panneau de contrôle relié au système de pilotage (5) reçoit les informations du ou des automates du système de pilotage (5) et affiche les alarmes destinées aux utilisateurs.

5. Installation selon au moins l'une des revendications précédentes, **caractérisée en ce que** les capteurs (12, 13) permettent de mesurer la pression instantanée régnant dans la ligne (11), en amont ou en aval du clapet anti-retour (15), et de transmettre ces mesures de pression sous forme de signaux électriques au système de pilotage (5).

6. Installation selon au moins l'une des revendications précédentes, **caractérisée en ce que** le dispositif anti-retour (15) comprend un clapet anti-retour.

7. Installation selon au moins l'une des revendications précédentes, **caractérisée en ce que** la première ligne annexe d'alimentation en gaz (21) comprend un dispositif de réduction de pression (33) agencé entre la bouteille (2) et la vanne pilotable (22).

8. Installation selon au moins l'une des revendications précédentes, **caractérisée en ce qu'**elle comporte une deuxième ligne annexe d'alimentation en gaz (41) reliée fluidiquement par son extrémité amont (41b) à une bouteille de gaz (2) et par son extrémité aval (41a) à la ligne principale de fluide (11), de préférence entre le moyen anti-retour (15) et l'orifice de sortie (14b).

9. Installation selon au moins l'une des revendications précédentes, **caractérisée en ce qu'**elle comporte une alarme sonore ou visuelle se déclenchant en réponse à une détection par l'un ou l'autre des capteurs (12, 13) d'une valeur de pression inférieure ou égale à une valeur de pression seuil au sein de la ligne principale de gaz (11).

10. Installation selon au moins l'une des revendications précédentes, **caractérisée en ce qu'**une découpe dans l'une des portes (160,161) de l'armoire permet à l'utilisateur d'avoir accès à différents afficheurs ou touches de commande, situés sur le boîtier (63) à panneau de contrôle.

11. Canalisation (50, 50') d'acheminement d'un gaz entre une source de gaz et au moins un site utilisateur, ladite source de gaz étant raccordée fluidiquement à une entrée de ladite canalisation (50, 50') de manière alimenter ladite canalisation (50, 50') avec ledit gaz, **caractérisée en ce que** ladite canalisation (50, 50') est raccordée en outre au moins à une entrée (14a) et à au moins une sortie (14b) d'une ligne principale (11) de gaz d'une installation selon l'une des revendications précédentes.

12. Canalisation (50, 50') selon la revendication 11, **caractérisée en ce que** l'installation selon l'une des revendications 1 à 11 est agencée sur ladite canalisation (50, 50') en aval d'une unité de détente (51) de gaz agencée sur ladite canalisation (50, 50').

## Patentansprüche

1. Gasausgabeanlage, umfassend:
- eine Hauptgasleitung (11), umfassend eine Gaseingangsöffnung (14a) und eine Gasausgangsöffnung (14b),
- eine erste Gasversorgungs-Zusatzleitung (21), die fluidisch über ein nachgelagertes Ende (21a) mit der Hauptfluidleitung (11) verbunden ist, und enthaltend mindestens ein kontrollierbares Ventil (22), das ausgelegt ist, um den Gasdurchgang von der Zusatzleitung (21) hin zur Hauptleitung (11) zu steuern,
- eine Gasrückschlageinrichtung (15), die auf der Hauptfluidleitung (11) angeordnet ist,
- einen ersten Drucksensor (12), enthaltend einen gasförmigen Druckanschluss, der fluidisch mit der Hauptleitung (11) zwischen der Eingangsöffnung (14a) und dem Rückschlagmittel (15) verbunden ist, und/oder einen zweiten Drucksensor (13), enthaltend einen Druckanschluss, der fluidisch mit der Hauptleitung (11) zwischen dem Rückschlagmittel (15) und der Ausgangsöffnung (14b) verbunden ist, und
- ein Kontrollsystem (5), das mit dem oder den ersten Drucksensor (12) und/oder zweiten Drucksensor (13) verbunden ist, und das das mindestens eine kontrollierbare Ventil (22) in Reaktion auf mindestens eine Druckmessung, durchgeführt von mindestens einem der Sensoren (12, 13), leitet,
**dadurch gekennzeichnet, dass**:
- das kontrollierbare Ventil (22) ein Elektroventil ist,
- das Kontrollsystem (5) mindestens einen programmierbaren Automaten enthält,
- mindestens ein Teil der Hauptfluidleitung (11), die Fluidversorgungs-Zusatzleitung (21), das kontrollierbare Ventil (22), die Rückschlageinrichtung, der erste Drucksensor (12), der zweite Drucksensor (13) und das Kontrollsystem (5) in einem Schrank (1) enthalten sind,
- und sie weiter Folgendes enthält:
- eine Bypassleitung (31), die fluidisch mit der ersten Zusatzversorgungsleitung (21) vorgelagert und nachgelagert vom kontrollierbaren Ventil (22) verbunden ist, wobei die Bypassleitung (31) ein manuelles Ventil (32) umfasst, das vom Benutzer betätigt werden kann, das normalerweise geschlossen ist, und
- ein Gehäuse (63) mit Steuertafel, die mit dem Kontrollsystem (5) verbunden ist, um einen spezifischen Alarm anzuzeigen, um den Benutzer für den Fall einer Fehlfunktion des Elektroventils (22) zu warnen und ihm zu ermöglichen, das manuelle Ventil (32) zu betätigen und manuell zu öffnen.

2. Anlage nach dem vorstehenden Anspruch, **dadurch gekennzeichnet, dass** sie weiter mindestens eine Gasflasche (2) enthält, die fluidisch mit einem vorgelagerten Ende (21b) der Gasversorgungs-Zusatzleitung (21) verbunden ist.

3. Anlage nach Anspruch 1, **dadurch gekennzeichnet, dass** das Kontrollsystem (5) ein Auslösen des spezifischen Alarms befiehlt, der den Benutzer im Fall einer Fehlfunktion des Elektroventils (22) warnt.

4. Anlage nach mindestens einem der vorstehenden Ansprüche, **dadurch gekennzeichnet, dass** das Gehäuse (63) mit Steuertafel, das mit dem Kontrollsystem (5) verbunden ist, die Informationen des oder der Automaten des Kontrollsystems (5) erhält und die Alarme, die für die Benutzer bestimmt sind, anzeigt.

5. Anlage nach mindestens einem der vorstehenden Ansprüche, **dadurch gekennzeichnet, dass** die Sensoren (12, 13) ermöglichen, den momentanen Druck, der in der Leitung (11) herrscht, vorgelagert und nachgelagert vom Rücklaufventil (15) zu messen und diese Druckmessungen in Form von elektrischen Signalen an das Kontrollsystem (5) zu übertragen.

6. Anlage nach mindestens einem der vorstehenden Ansprüche, **dadurch gekennzeichnet, dass** die Rückschlageinrichtung (15) ein Rückschlagventil umfasst.

7. Anlage nach mindestens einem der vorstehenden Ansprüche, **dadurch gekennzeichnet, dass** die erste Gasversorgungs-Zusatzleitung (21), eine Druckminderungseinrichtung (33) umfasst, die zwischen der Flasche (2) und dem kontrollierbaren Ventil (22) angeordnet ist.

8. Anlage nach mindestens einem der vorstehenden Ansprüche, **dadurch gekennzeichnet, dass** sie eine zweite Gasversorgungs-Zusatzleitung (41) enthält, die fluidisch durch ihr vorgelagertes Ende (41b) mit einer Gasflasche (2) und durch ihr nachgelagertes Ende (41a) mit der Hauptfluidleitung (11) verbunden ist, vorzugsweise zwischen dem Rückschlagmittel (15) und der Ausgangsöffnung (14b).

9. Anlage nach mindestens einem der vorstehenden Ansprüche, **dadurch gekennzeichnet, dass** sie einen akustischen oder visuellen Alarm enthält, der in Reaktion auf einen Nachweis durch den einen oder den anderen der Sensoren (12, 13) eines Druckwerts von weniger oder gleich einem Schwellendruckwert innerhalb der Hauptgasleitung (11) ausgelöst wird.

10. Anlage nach mindestens einem der vorstehenden Ansprüche, **dadurch gekennzeichnet, dass** ein Ausschnitt in einer der Türen (160,161) des Schranks dem Benutzer ermöglicht, Zugang zu verschiedenen Anzeigen oder Befehlstasten zu haben, die sich auf dem Gehäuse (63) mit Steuertafel befinden.

11. Rohrleitung (50, 50') zur Führung eines Gases zwischen einer Gasquelle und mindestens einer Benutzerstelle, wobei die Gasquelle mit einem Eingang der Rohrleitung (50, 50') fluidisch verbunden ist, um die Rohrleitung (50, 50') mit dem Gas zu versorgen, **dadurch gekennzeichnet, dass** die Rohrleitung (50, 50') weiter mit mindestens einem Eingang (14a) und mindestens einen Ausgang (14b) einer Hauptgasleitung (11) einer Anlage nach einem der vorstehenden Ansprüche verbunden ist.

12. Rohrleitung (50, 50') nach Anspruch 11, **dadurch gekennzeichnet, dass** die Anlage nach einem der Ansprüche 1 bis 11 auf der Rohrleitung (50, 50') nachgelagert von einer Gasexpansionseinheit (51) angeordnet ist, die auf der Rohrleitung (50, 50') angeordnet ist.

## Claims

1. Gas dispensing unit comprising:
- a main gas line (11) comprising an inlet orifice (14a) and an outlet orifice (14b) for the gas,
- a first auxiliary gas supply line (21) fluidly connected, via a downstream end (21a), to the main fluid line (11), and comprising at least one controllable valve (22) capable of controlling the passage of gas from the auxiliary line (21) to said main line (11),
- a gas non-return device (15) arranged on the main fluid line (11),
- a first pressure sensor (12) comprising a gas pressure port fluidly connected to the main line (11) between the inlet (14a) and the non-return means (15), and/or a second pressure sensor (13) comprising a gas pressure port fluidly connected to the main line (11) between the non-return means (15) and the outlet orifice (14b), and
- a control system (5) connected to said first pressure sensor (12) and/or second pressure sensor (13) and controlling said at least one controllable valve (22) in response to at least one pressure measurement taken by at least one of said sensors (12, 13),
**characterised in that**:
- the controllable valve (22) is a solenoid valve,
- the control system (5) comprises at least one programmable controller,
- at least one portion of the main fluid line (11), the auxiliary fluid supply line (21), the controllable valve (22), the non-return device, the first pressure sensor (12), the second pressure sensor (13) and the control system (5) are comprised in a cabinet (1),
- and it further comprises:
- a bypass line (31) fluidically connected to the first auxiliary supply line (21), upstream and downstream of the controllable valve (22), said bypass line (31) comprising a manual valve (32) that can be manoeuvred by the user and that is, normally, closed, and
- a case (63) with a control panel connected to the control system (5), in order to display a specific alarm in order to warn the user, in case of malfunction of the solenoid valve (22), and allow the user to actuate and manually open the manual valve (32).

2. Unit according to the preceding claim, **characterised in that** it further comprises at least one gas cylinder (2) fluidly connected to an upstream end (21b) of the auxiliary gas supply line (21).

3. Unit according to claim 1, **characterised in that** the control system (5) controls a triggering of the specific alarm that warns the user in case of a malfunction of the solenoid valve (22).

4. Unit according to at least any one of the preceding claims, **characterised in that** the case (63) with a control panel connected to the control system (5) receives the information from the controller or controllers of the control system (5) and displays the alarms intended for the users.

5. Unit according to at least any one of the preceding claims, **characterised in that** the sensors (12, 13) make it possible to measure the instantaneous pressure present in the line (11), upstream or downstream from the non-return valve (15), and to transmit these pressure measurements in the form of electrical signals to the control system (5).

6. Unit according to at least any one of the preceding claims, **characterised in that** the non-return device (15) comprises a non-return valve.

7. Unit according to at least any one of the preceding claims, **characterised in that** the first auxiliary gas supply line (21) comprises a pressure reducing device (33) arranged between the cylinder (2) and the controllable valve (22).

8. Unit according to at least any one of the preceding claims, **characterised in that** it comprises a second auxiliary gas supply line (41) fluidly connected by its upstream end (41b) to a gas cylinder (2) and by its downstream end (41a) to the main fluid line (11), preferably between the non-return means (15) and the outlet orifice (14b).

9. Unit according to at least any one of the preceding claims, **characterised in that** it comprises an audible or visual alarm that is triggered in response to a detection by one or the other of the sensors (12, 13) of a pressure value less than or equal to a threshold pressure value within the main gas line (11).

10. Unit according to at least any one of the preceding claims, **characterised in that** a cut in one of the doors (160,161) of the cabinet allows the user to have access to the various displays or control buttons, located on the case (63) for a control panel.

11. Pipeline (50, 50') for conveying a gas between a source of gas and at least one user site, said source of gas being fluidly connected to an inlet of said pipeline (50, 50') in such a way as to supply said pipeline (50, 50') with said gas, **characterised in that** said pipeline (50, 50') is further connected at least to an inlet (14a) and to at least one outlet (14b) of a main gas line (11) of a unit according to any one of the preceding claims.

12. Pipeline (50, 50') according to claim 11, **characterised in that** the unit according to any of claims 1 to 11 is arranged on said pipeline (50, 50') downstream from a gas expansion unit (51) arranged on said pipeline (50, 50').
